# EUROPÄISCHE PATENTSCHRIFT

(11) **EP 2 040 664 B1**
(45) Veröffentlichungstag und Bekanntmachung des Hinweises auf die Patenterteilung: **28.10.2009**
(21) Anmeldenummer: 07765708.8
(22) Anmeldetag: 28.06.2007
(51) Int. Cl.: A61K 8/14, A61K 8/97, A61K 8/67, A61Q 19/08

(54) **KOSMETISCHE ZUBEREITUNG MIT EINEM HAUTPFLEGEKOMPLEX MIT ANTI-ALTERUNGSWIRKUNG**
COSMETIC PREPARATION COMPRISING AN ANTI-AGING SKIN CARE COMPLEX
PRÉPARATION COSMÉTIQUE CONTENANT UN COMPLEXE DE SOIN POUR LA PEAU À ACTION ANTI-VIEILLISSEMENT

(30) Priorität: 05.07.2006 DE 102006031762
(43) Veröffentlichungstag der Anmeldung: 01.04.2009
(73) Patentinhaber: Coty Prestige Lancaster Group GmbH, 55116 Mainz (DE)
(72) Erfinder: GOLZ-BERNER, Karin, 98000 Monaco (MC); ZASTROW, Leonhard, 98000 Monaco (MC)
(74) Vertreter: Gulde Hengelhaupt Ziebig & Schneider
(86) Internationale Anmeldenummer: PCT/EP2007/056494
(87) Internationale Veröffentlichungsnummer: WO 2008/003638

(56) Entgegenhaltungen:
- EP-A2- 0 826 366
- WO-A-03/013562
- WO-A-2006/048158

## Beschreibung

Die Erfindung betrifft eine kosmetische Zubereitung mit einem Hautpflegekomplex mit Anti-Alterungswirkung, der aus Liposomen besteht, die ein Gemisch von kosmetischem Öl, Extrakt der Samen von Plukenetia volubilis, Extrakt der Blätter von Cynara scolymus und ein hydriertes Retinol umfassen, und die Liposomen homogen in einem aus Wasser und einem Gelbildner bestehenden Gelnetzwerk verteilt sind, und das Gel in einer Konzentration von 0,1-11 Gew-% zusammen mit üblichen kosmetischen Zusatzstoffen, Hilfsstoffen, Wirkstoffen und Gemischen davon in der kosmetischen Zubereitung enthalten ist, wobei die Konzentration auf das Gesamtgewicht der Zubereitung bezogen ist.

Eine Vielzahl kosmetischer Erzeugnisse mit Anti-Alterungswirkung ist bereits bekannt. Die WO-A-2006/048158 offenbart beispielsweise, dass Pflanzen- und Samenextrakte aus Plukenetia volubilis sowie die daraus isolierten Proteine und Proteingemische eine Anti-Alterungswirkung auf die Haut aufweisen. Dabei wird bereits nach 10 min ein straffender Effekt erzielt.

Ein wesentlicher Bestandteil vieler bekannter Erzeugnisse ist Retinol oder ein Derivat davon, die Retinoinsäure, Retinylpalmitat, Tocopherolretinoat, C₂-C₅-Ester von Retinol und andere. Die Wirkung dieser Erzeugnisse ist jedoch nicht immer befriedigend, da oftmals die Stabilität nicht ausreichend ist und damit die Wirksamkeit schnell verringert wird und darüber hinaus Hautirritationen auftreten können.

EP 826 366 beschreibt die Verringerung der Irritationswirkung von Hydroxysäuren oder Retinol bzw. Retinoiden durch den Zusatz von Borretschsamenöl in wässrigen Systemen.

Artischockenblätter (cynara scolymus) sind bisher weitgehend nur in Nahrungsmitteln eingesetzt worden, teilweise auch zusammen mit anderen Pflanzenprodukten in kosmetischen Haarprodukten.

Die in WO 99/61035 beschriebene Verwendung von Artischockenpflanzen betrifft ein oral einzunehmendes pharmazeutisches Präparat zur Behandlung von Hyperlipidämie. Die WO-A-03/013562 offenbart, dass durch Variation des Gehaltes von Mono- und Di- Caffeoylchinasäuren (CCS) und Flavonoiden das Wirkungsspektrum von Artischockenblätterextrakten variiert werden kann. Die hergestellten Extrakte werden zur Herstellung von kosmetischen Zubereitungen mit antioxidativer, zell- und organoprotektiver Wirkung vorgeschlagen.

Der Erfindung liegt die Aufgabe z-ugrunde, ein Hautpflegekosmetikum mit Anti-Alterungswirkung bereitzustellen, das eine schnelle Wirksamkeit zeigt, die Wirkung über einen langen Zeitraum aufrechterhält und keine hautirritierende Nebenwirkung aufweist.

Erfindungsgemäß bereitgestellt wird eine kosmetische Zubereitung mit einem Hautpflegekomplex mit Anti-Alterungswirkung, worin der Komplex aus Liposomen besteht, die ein Gemisch von kosmetischem Öl, Extrakt der Samen von Plukenetia volubilis, Extrakt der Blätter von Cynara scolymus und einem hydrierten Retinol umfassen, und die Liposomen homogen in einem aus Wasser und einem Gelbildner bestehenden Gelnetzwerk verteilt sind, und dieser Komplex in Form des Gels in einer Konzentration von 0,1-11 Gew-% zusammen mit üblichen kosmetischen Zusatzstoffen, Hilfsstoffen, Wirkstoffen und Gemischen davon in der kosmetischen Zubereitung enthalten ist. Die Konzentrationsangaben sind auf das Gesamtgewicht der Zubereitung bezogen.

Besonders bevorzugt sind Konzentrationen des Gels, d.h. des Komplexes, in der Zubereitung im Bereich von 1 bis 6 Gew-%.

Es wurde gefunden, dass die Kombination von Inca inchi Samenextrakt (Plukenetia volubilis), Artischockenblattextrakt (Cynara scolymus) und hydriertem Retinol nicht nur besonders schnell wirksam ist, sondern die Anti-Alterungswirkung auch über einen langen Zeitraum aufrechterhält. Wesentlich für die schnelle und hohe Anfangswirksamkeit ist dabei die Einbettung der Liposome in ein Gelnetzwerk, worin die voneinander isolierten Liposome erst bei Kontakt mit der Haut infolge unterschiedlicher elektrischer Ladung von Haut/Liposom zerstört werden, die Wirkstoffe freigeben, und diese ihre volle Wirksamkeit sofort entfalten können.

Der Mechanismus für die langanhaltende Wirksamkeit ist noch nicht geklärt, beruht jedoch vermutlich auf einem synergistischen Effekt der beiden Pflanzenextrakte mit dem hydrierten Retinol. Die Wirksamkeit ist besonders signifikant bei reifer Haut von Anwendern, die älter als 50 Jahre sind. Insbesondere bei dieser Haut wurde gefunden, dass Faltenanzahl und Faltentiefe deutlich reduziert wurden, wie dies in den Beispielen dargestellt wird.

Die mit Wasser und den eingesetzten Ölen gebildeten Liposomen sind insbesondere Phospholipidliposomen, die aus dem Stand der Technik bekannt sind. Dabei handelt es sich um vollständig geschlossene Lipid-Bilayer-Membranen, die ein wässriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wässrige Schicht getrennt ist). Die Bilayer besteht aus zwei Lipid-Monolayern, die einen hydrophoben "Schwanz"-Bereich und einen hydrophilen "Kopf"-Bereich haben. Die Struktur der Membran-Bilayer ist so, dass die hydrophoben (unpolaren) "Schwänze" der Lipidmonolayer sich in Richtung des Zentrums der Bilayer orientieren, während sich die hydrophilen "Köpfe" in Richtung der wässrigen Phase orientieren.

Die Herstellung von Liposomen, aus gesättigten und ungesättigten Lipiden, ist in sehr vielen Patenten beschrieben worden, ebenso deren Einsatz als Transportsystem. Die Einarbeitung des erfindungsgemäßen Hautpflegekomplexes kann auf übliche Weise erfolgen.

Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon® oder NAT®.

Das die Liposomen aufnehmende Gel ist vorzugsweise ein solches, das aus einem Acrylatpolymeren besteht, z.B. Pemulen TR1® Polymeric Emulsion (INCI: Acrylates/C10-30 Alkyl Acrylate Crosspolymer), das hier als Gelbildner wirkt, d.h. mit Wasser ein hochwirksames wässriges Mikrogel ausbildet, das die Öltröpfchen voneinander isoliert hält. Dabei werden die hydrophoben Teile des Polymers in der Ölphase verankert.

Der in dem Hautpflegekomplex eingesetzte Extrakt der Samen von Inca inchi (Plukenetia volubilis) ist ein Produkt, das hergestellt wird durch Kaltpressen der Samen und anschließender Reinigung durch Dekantieren und Filtration.

Der Anteil des Inca inchi-Extraktes beträgt 1,0 bis 3 Gew.-%, bezogen auf die Zusammensetzung des Komplexes.

Der in dem Hautpflegekomplex eingesetzte Extrakt von Artischockenblättern (Cynara scolymus) ist ein bei Umgebungstemperatur (15-25 °C) gewonnener Extrakt mit Propylenglycol.

Der Anteil des Artischockenblätterextraktes beträgt 2 bis 4 Gew.-%, bezogen auf die Zusammensetzung des Komplexes.

Das verwendete hydrierte Retinol ist ein Retinol, bei dem alle Doppelbindungen durch Hydrierung gesättigt wurden. Dieses Produkt zeigt eine verbesserte Photo- und Wärmestabilität sowie weniger Hautirritation als natives Retinol. Es liegt vorzugsweise in einer 10 %igen Zubereitung mit Caprylic/Capric Acid Triglyceride vor und wird in dieser Form eingesetzt.

Der Anteil des hydrierten Retinols in der bevorzugten Zubereitung beträgt 0,1 bis 1 Gew.-%, bezogen auf die Zusammensetzung des Komplexes.

Der Komplex besteht im allgemeinen aus Wasser, 3 bis 10 Gew.-% Phospholipiden, 0,3 bis 1 Gew.-% des Gelbildners, 3 bis 10 Gew.-% einwertiger Alkohol (z. B. Ethanol), 5 bis 12 Gew.-% mehrwertiger Alkohol (z.B. Glycerin), den Wirkstoffen Inca inchi-Extrakt 1 bis 3 Gew.-%, Artischockenextrakt 2 bis 4 Gew.-%, hydriertes Retinol 0,1 bis 1 Gew.-% und weiteren Hilfsstoffen wie Konservierungsmitteln, TEA, Ölen usw.

Das Hautpflege-Kosmetikum kann als Zubereitung weiterhin bekannte Hilfs- und Trägerstoffe, wie sie üblicherweise in solchen Zubereitungen verwendet werden, enthalten, z.B. Wasser, Konservierungsmittel, Farbstoffe, Pigmente mit färbender Wirkung, Verdickungsmittel, Duftstoffe, Alkohole, Polyole, Öle, Ester, Elektrolyte, Gelbildner, Copolymere, Siliconemulgatoren, Wachse, Stabilisatoren und Gemische davon.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind pflanzliche Öle mit hohen Anteilen an Omega-3- und Omega-6-Fettsäuren, beispielsweise Nussöle wie Haselnussöl. Auch andere pflanzliche Öle können eingesetzt werden, wie Calendulaöl, Jojobaöl, Avocadoöl, Macadamianussöl, Rizinusöl, Kakaobutter, Kokosnussöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinussöl, Distelöl und Gemische davon. Diese anderen Öle tragen dann aber nicht so gut zu der angestrebten Wirkungsrichtung bei.

Das Kosmetikum kann auch weitere Wirkstoffe enthalten. Dazu gehören z.B. anorganische und organische Lichtschutzmittel, Selbstbräunungsmittel, Radikalfänger, Vitamine, Enzyme, pflanzliche Wirkstoffe, Polymere, Antioxidationsmittel sowie Omega-3-/6-Fettsäuren.

Zu Antioxidationsmitteln gehören Vitamine wie Vitamin C und Derivate davon, beispielsweise Ascorbylacetat, -phosphat und -palmitat, Magnesiumascorbylphosphat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B. α-Carotin, β-Carotin, Lycopin; Harnsäure und Derivate davon; α-Hydroxysäuren wie Citronensäure, Milchsäure, Apfelsäure; α-Hydroxyfettsäuren wie Palmitinsäure, Phytinsäure, Lactoferrin; Stilbene und deren Derivate; Mannose und deren Derivate; Liponsäure und deren Derivate, z.B. Dihydroliponsäure; Ferulasäure und deren Derivate; Thiole wie Glutathion, Cystein, Cystin und deren Ester; Folsäure und deren Derivate.

Bevorzugte Antioxidationsmittel bzw. Radikalfänger sind Pflanzenextrakte, insbesondere solche, die aus der Gruppe ausgewählt sind, bestehend aus Angelica archangelica, Arnica montana, Camellia sinensis, Cupressus semper, Coffee arabica, Polygonatum multiflorum, Pongamia pinnata, Rosmarinus officinalis, Evernia fufuracea, Evernia prunastri, Aventa sativa und Gemische davon.

Als Radikalfänger sind speziell bevorzugt Vitamin A, Vitamin E, Peptide, Flavone, Flavonole sowie Pflanzenextrakte. Ein besonders vorteilhafter Pflanzenextrakt ist ein solcher aus getrockneten Extrakten von Angelica archangelica, Camellia sinensis, Coffee arabica und Pongamia pinnata in einem einwertigen Alkohol.

Dem erfindungsgemäßen Kosmetikum können auch entsprechende wasser- und/oder öllösliche UVA- oder UVB-Filter oder beide zugesetzt werden, allerdings vorzugsweise nur bis zur Erreichung eines Sonnenschutzfaktors SPF 8 (Bestimmung gemäß Colipa-Standard (Colipa-Ref.: 94/289 (1994)). Zu vorteilhaften öllöslichen UVB-Filtern gehören 4-Aminobenzoesäure-Derivate wie der 4-(Dimethylamino)-benzoesäure-(2-ethyl-hexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Dem Kosmetikum zugesetzte Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titandioxid, Glimmer, Kaolin, manganhaltige Tone, Calciumcarbonat, Talkum, Glimmer-Titanoxid, Glimmer-Titanoxid-Eisenoxid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Als weitere Wirkstoffe geeignete Substanzen sind Selbstbräunungsmittel, wie z.B. Isatin, Alloxan, Ninhydrin, Glycerinaldehyd, meso-Weinsäurealdehyd, Glutaraldehyd, Erythrulose, Pirazolin-4,5-dionderivate, Dihydroxyaceton (DHA), 4,4-Dihydroxypirazolin-5-dionderivate.

Ein weiterer Zusatz für das erfindungsgemäße Kosmetikum ist eine Wirkstoffzubereitung mit hohem Radikalschutzfaktor gemäß WO 99/66881 mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Besonders bevorzugt als zusätzlicher Wirkstoff ist auch eine Zubereitung mit Pflanzenextrakten gemäß WO 2004/105704, die Pflanzenextrakte auf alkoholischer Basis enthält, bestehend aus 0,1 bis 2 Gew-% Extrakt grüner Kaffebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen C₂-C₅-Alkohol, wobei der Radikalschutz-faktor im Bereich von 1400-2900 × 10¹⁴ Radikale pro mg liegt.

Weiterhin bevorzugt als zusätzliche Wirkstoffe sind Omega-3-Fettsäuren, Omega-6-Fettsäuren oder Gemische davon. Zu Omega-3-Fettsäuren gehören Alpha-Linolensäure, Eicosapentaensäure und Docosahexaensäure. Zu Omega-6-Fettsäuren gehören Linolsäure, Arachidonsäure und Gamma-Linolensäure. Diese Fettsäuren sind sog. essentielle Fettsäuren für die Ernährung des Menschen, können aber zusammen mit den Wirkstoffen der vorliegenden Erfindung die anti-age-Wirkung auf der Haut noch verbessern. Gehalten von 1 bis 6 Gew.-% sind bevorzugt, insbesondere 2 bis 4,5 Gew.-%.

Die Erfindung betrifft auch ein Herstellungsverfahren des speziellen Hautpflegekomplexes und damit der Zubereitung. Erfindungsgemäß werden
a) ein oder mehrere Öle sowie die Wirkstoffe Inca inchi-Extrakt, Artischockenextrakt und hydriertes Retinol mit Alkohol und Wasser bei 15-25 °C zu einem ersten Gemisch vermischt; anschließend wird
b) das unter a) hergestellte erste Gemisch zu einem separat hergestellten zweiten Gemisch gegeben, wobei das zweite Gemisch aus einem Phospholipid und ggf. weiteren Hilfs- und Wirkstoffen besteht mit einer Zugabegeschwindigkeit von nicht mehr als 10 g/min bei 1000-3000 U/min;
c) das dabei entstandene dritte Gemisch wird bis zur Bildung der Liposomen bei 600-800 U/min gerührt;
d) Wasser und der Geldbildner werden zusammengegeben und bis zur Ausbildung eines Gelnetzwerkes bei nicht mehr als 1200 U/min gerührt; und
e) das dritte Gemisch wird bei weniger als 700 U/min über einen Zeitraum von 15-40 min in das Gel gemäß Stufe d) eingebracht;
f) das gemäß e) erhaltene Gel bei 25 bis 35°C und weniger als 700 U/min mit kosmetischen Hilfsstoffen, Wirkstoffen oder Gemischen davon vermischt.

Die erfindungsgemäße Zubereitung kann alle üblichen kosmetischen Formen haben, z.B. Tagescreme, Nachtcreme, Sonnengel, Masken, Körperlotionen, Reinigungsmilch, Make up, Cremereiniger, Lippenstifte, Körperpuder, Augenkosmetik, Haarmasken, Haarspülungen, Haarshampoos, Duschgele, Duschöl, Badeöl, Deo-Stifte, Parfüm-Stifte, Kühl-Roller, Deo-Roller, Kompaktpuder oder Kompaktwachs gegebenenfalls mit Applikator, Rouge, Grundierung, Sonnenschutzzubereitung (Sun care oder After sun).

Die Erfindung soll nachstehend durch Beispiele näher erläutert werden. Alle Angaben erfolgen in Gewichtsprozent, sofern nichts anderes angegeben ist.

### Beispiel 1 Herstellung des Hautpflegekomplexes 50+

| **Phase A** | % |
|---|---|
| Lecithine | 5-8 |
| Glycerine | 8-10 |
| Wheat Germ Oil | 1-3 |

| **Phase B** | |
|---|---|
| Wasser | 40 |
| Alcohol | 5-7 |
| Hazelnut Oil | 2-5 |
| Inca inchi Seed Extract | 1-3 |
| Artichoke Leaf Extract (in PPG) | 2-4 |
| Retinol Hydrogenated | 0,1-1 |
| Konservierungsmittel | 0,5-1 |

| **Phase D** | |
|---|---|
| Wasser | q.s. ad 100 |
| Acrylates/C10-C30 Alkyl Acrylate Crosspolymer | 0,3-0,5 |
| Triethanol Amine | 0,3-0,5 |
| Konservierungsmittel | 0,5 |

Die Phase A wird nach Zugabe aller Bestandteile bei wenigstens 2000 U/min ca. 40-60 Minuten bei 20-40 °C homogenisiert. Separat werden die Bestandteile der Phase bei 500-600 U/min zusammengegeben und 20 Minuten gerührt. Die Phase B wird zur Phase A sehr langsam gegeben (ca. 1 Stunde für insgesamt 1 kg Gemisch) unter Rühren bei ca. 3000 U/min und bei Temperaturen unter 40 °C), und beide bilden die Liposome der Phase C, die bei ca. 600-800 U/min gerührt werden.

Separat wird der Gelbildner von Phase D in Wasser gegeben und bei ca. 1000 U/min für 10 Minuten gerührt bis zur Ausbildung des Gelnetzwerkes. Danach wird die Phase C zur Phase D gegeben bei etwa 600 U/min für 30 Minuten, um ca. 1 kg des Produktes (Komplex 50+) zu erhalten.

### Beispiel 2 Kosmetische Zubereitung als Tagescreme

| **Phase A** | |
|---|---|
| Wasser | q.s. ad 100 |
| Disodium EDTA | 0,1 |
| Propylenglycole | 2 |
| Glycerine | 5 |

| **Phase B** | |
|---|---|
| Chlorphenesin | 0,5 |
| Diisopropyl Sebacate | 9 |
| Cetearyl Alcohol (75°C) | 2 |
| Cyclohexasiloxane & Cyclopentasiloxane | 3 |

| **Phase C** | |
|---|---|
| Phase C wird aus Phase A und B gebildet, die bei 75 °C zusammengegeben, 5 Minuten homogenisiert werden bei ca. 3000 U/min und auf 35 °C abgekühlt werden. | |

| **Phase D** | |
|---|---|
| Homogenisierungsmittel | 0,3 |
| Parfüm | 0,5 |
| Komplex 50+ von Beispiel 1 | 10 |

(Inca inchi 1,5%, Artischoke 2,5%, Retinol hydr. 0,4%, bezogen auf das Gesamtgewicht des Komplexes)

Die Phase D wird mit Phase C bei 20-35 °C und weniger als 1000 U/min vermischt.

### Beispiel 3 Kosmetische Zubereitung als Augencreme

| **Phase A** | |
|---|---|
| Wasser | q.s. ad 100 |
| EDTA | 0,3 |
| Glycerine | 5 |

| **Phase B** | |
|---|---|
| PEG-100 Stearate Glyeryl Stearate | 4 |
| Shea Butter | 2 |
| Cetearyl Alcohol (70°C) | 3,5 |
| Cyclopentasiloxane & Dimethiconol | 5 |

| **Phase C** | |
|---|---|
| Phase C wird aus Phasen A und B gebildet, die bei 75 °C zusammengegeben, 5 Minuten homogenisiert werden bei ca. 3000 U/min und auf 35 °C abgekühlt werden. | |

| **Phase D** | |
|---|---|
| Parfüm | 0,5 |
| RPF-Komplex* | 1 |
| Komplex 50+ von Beispiel 1 und 2 | 5 |
| Konservierungsmittel | 0,3 |
| Herstellung wie im Beispiel 2. | |

### Beispiel 4 Kosmetische Zubereitung als Cremereiniger

| **Phase A** | |
|---|---|
| Wasser | q.s. ad 100 |
| EDTA | 0,1 |
| Propylenglycole | 3,5 |
| Glycerine | 3 |

| **Phase B** | |
|---|---|
| Steareth-2 | 2,5 |
| Steareth-21 | 2 |
| Glyceryl Stearate | 2 |
| Dimethicone | 5 |

| **Phase C** | |
|---|---|
| Phase C wird aus Phasen A und B gebildet, die bei 75 °C zusammengegeben, 5 Minuten homogenisiert werden bei ca. 3000 U/min und auf 35 °C abgekühlt werden. | |

| **Phase D** | |
|---|---|
| Parfüm | 0,3 |
| RPF-Komplex* | 0,1 |
| Komplex 50+ von Beispiel 1 und 2 | 0,1 |
| Konservierungsmittel | 0,3 |

| | |
|---|---|
| * gemäß Beispiel 1 von WO WO2004/105706. | |

### Herstellung wie im Beispiel 2.

### Beispiel 5 Anwender-Test

Es wurde eine Formulierung gemäß Beispiel 2 hergestellt (A). Eine weitere Formulierung gemäß Beispiel 2, bei der Artischockenextrakt und Retinol fehlte, wurde mit (B) bezeichnet.

Eine weitere Formulierung gemäß Beispiel 2 mit üblichem Retinol, bei der Artischockenextrakt und Inca inchi fehlte, wurde mit (C) bezeichnet.

Eine weitere Formulierung gemäß Beispiel 2, bei der Inca inchi und Retinol fehlte, wurde mit (D) bezeichnet.

Der Test wurde mit 15 Teilnehmerinnen im Alter von 52 bis 66 Jahren durchgeführt. Die Teilnehmerinnen applizierten zweimal täglich die Cremes A, B, C und D auf vier markierten Arealen des linken und rechten Unterarms. In den vorgegebenen Abständen von 2 Wochen wurden digitalisierte Aufnahmen von den markierten Stellen gemacht und über ein Computerprogramm nach Anzahl der Falten und Faltentiefe ausgewertet.

Die Ergebnisse sind in Tabelle 1 dargestellt.

**Tabelle 1**

| | Faltenreduzierung [%] | | | |
|---|---|---|---|---|
| | A | B | C | D |
| Behandlungsbeginn | 0 | 0 | 0 | 0 |
| nach 2 Wochen | 19-21 | 5-7 | 7-11 | 4-6 |
| 4 Wochen | 27-29 | 9-10 | 7-11 | 4-7 |
| 6 Wochen | 33-35 | 11-12 | 10-14 | 8-9 |
| 8 Wochen | 39-40 | 11-12 | 15-19 | 6-7 |
| 12 Wochen | 40-42 | 10-12 | 16-18 | 7-8 |

Tabelle 1 zeigt mit der Zubereitung A eine überlegene Wirkung, die auf einen Synergismus schließen lässt. Auch gegenüber der alleinigen Verwendung von Retinol (Zubereitung C) ist die Wirkung deutlich besser. Darüber hinaus hält die Wirkung bei der erfindungsgemäßen Zubereitung A über einen langen Zeitraum (12 Wochen) an.

### Beispiel 6

Es wurde eine Tagescreme gemäß Beispiel 2 hergestellt, wobei die Phase B zusätzlich 3 % Omega-3-/6-Fettsäuren (Gemisch aus alpha-Linolensäure, Linolsäure und Arachidonsäure) enthielt. Im Anwendertest gemäß Beispiel 5 zeigte sich eine Faltenreduzierung nach 8 Wochen von 42 % und nach 12 Wochen von 43,5 %.

## Patentansprüche

1. Kosmetische Zubereitung mit einem Hautpflegekomplex mit Anti-Alterungswirkung, **dadurch gekennzeichnet, dass** der Komplex aus Liposomen besteht, die ein Gemisch von kosmetischem Öl, Extrakt der Samen von Plukenetia volubilis, Extrakt der Blätter von Cynara scolymus und ein hydriertes Retinol umfassen, und die Liposomen homogen in einem aus Wasser und einem Gelbildner bestehenden Gelnetzwerk verteilt sind, und das Gel in einer Konzentration von 0,1-11 Gew-% zusammen mit üblichen kosmetischen Zusatzstoffen, Hilfsstoffen, Wirkstoffen und Gemischen davon in der kosmetischen Zubereitung enthalten ist, wobei die Konzentration auf das Gesamtgewicht der Zubereitung bezogen ist.

2. Kosmetische Zubereitung nach Anspruch 1, worin der Extrakt der Samen von Plukenetia volubilis im Bereich von 1-3 Gew-% liegt, bezogen auf das Gesamtgewicht des Komplexes.

3. Kosmetische Zubereitung nach Anspruch 1, worin der Extrakt der Blätter von Cynara scolymus im Bereich von 2-4 Gew-% liegt, bezogen auf das Gesamtgewicht des Komplexes.

4. Kosmetische Zubereitung nach Anspruch 1, worin der Anteil an hydriertem Retinol im Bereich von 0,1-1 Gew-% liegt, bezogen auf das Gesamtgewicht des Komplexes.

5. Kosmetische Zubereitung nach Anspruch 1, worin der Anteil des Komplexes im Bereich von 1 bis 6 Gew-% liegt, bezogen auf das Gesamtgewicht der Zubereitung.

6. Kosmetische Zubereitung nach Anspruch 1, **dadurch gekennzeichnet, dass** als weitere Wirkstoffe Omega-3-/6-Fettsäuren enthalten sind.

7. Verfahren zur Herstellung einer kosmetischen Zubereitung mit einem Hautpflegekomplex mit Anti-Alterungswirkung, **dadurch gekennzeichnet, dass**
a) ein oder mehrere Öle sowie die Wirkstoffe Inca inchi-Extrakt, Artischockenextrakt und hydriertes Retinol mit Alkohol und Wasser bei 15-25 °C zu einem ersten Gemisch vermischt; anschließend wird
b) das unter a) hergestellte erste Gemisch zu einem separat hergestellten zweiten Gemisch gegeben, wobei das zweite Gemisch aus einem Phospholipid und weiteren Hilfs- und Wirkstoffen besteht mit einer Zugabegeschwindigkeit von nicht mehr als 10 g/min bei 1000-3000 U/min;
c) das dabei entstandene dritte Gemisch wird bis zur Bildung der Liposomen bei 600-800 U/min gerührt;
d) Wasser und der Geldbildner werden zusammengegeben und bis zur Ausbildung eines Gelnetzwerkes bei nicht mehr als 1200 U/min gerührt;
e) das dritte Gemisch wird bei weniger als 700 U/min über einen Zeitraum von 15-40 min in das Gel gemäß Stufe d) eingebracht; und
f) das gemäß e) erhaltene Produkt wird bei 20-35 °C und weniger als 700 U/min mit üblichen kosmetischen Hilfsstoffen, Wirkstoffen oder Gemischen davon vermischt.

## Claims

1. A cosmetic preparation with a skin care complex with anti-ageing effect, **characterized in that** said complex consists of liposomes comprising a mixture of cosmetic oil, extract of Plukenetia volubilis seeds, extract of Cynara scolymus leaves and hydrogenated retinol, said liposomes are homogeneously dispersed in a gel network consisting of water and a gel-forming agent, and the gel, together with common cosmetic additives, adjuvants, active substances and mixtures thereof, is included in the cosmetic preparation at a concentration of 0.1-11 wt.%, the concentration being based on the overall weight of the preparation.

2. The cosmetic preparation according to claim 1, wherein the extract of the Plukenetia volubilis seeds is in the range of 1-3 wt.%, relative to the overall weight of the complex.

3. The cosmetic preparation according to claim 1, wherein the extract of the Cynara scolymus leaves is in the range of 2-4 wt.%, relative to the overall weight of the complex.

4. The cosmetic preparation according to claim 1, wherein the share of hydrogenated retinol is in the range of 0.1-1 wt.%, relative to the overall weight of the complex.

5. The cosmetic preparation according to claim 1, wherein the share of the complex is in the range of 1-6 wt.%, relative to the overall weight of the preparation.

6. The cosmetic preparation according to claim 1, **characterized in that** omega-3/6 fatty acids are included as further active substances.

7. A method of producing a cosmetic preparation with a skin care complex with anti-ageing effect, **characterized in that**
a) one or more oils and the active ingredients Inca inchi extract, artichoke extract and hydrogenated retinol are mixed with alcohol and water at 15-25°C to form a first mixture; subsequently,
b) the first mixture produced under a) is added to a separately produced second mixture at an addition rate of no more than 10 g/min at 1,000-3,000 rpm, said second mixture consisting of a phospholipid and further adjuvants and active substances;
c) the third mixture thus formed is stirred at 600-800 rpm until the liposomes are formed;
d) water and the gel-forming agent are combined and stirred at no more than 1,200 rpm until a gel network is formed;
e) the third mixture is introduced into the gel of step d) at less than 700 rpm over a period of 15-40 min; and
f) the product obtained according to e) is mixed with common cosmetic adjuvants, active substances or mixtures thereof at 20-35°C and less than 700 rpm.

## Revendications

1. Préparation cosmétique comprenant un complexe de soins de la peau ayant un effet anti-vieillissement, **caractérisée en ce que** le complexe est constitué de liposomes comprenant un mélange d'huile cosmétique, d'extrait de graines de Plukenetia volubilis, d'extrait de feuilles de Cynara scolymus et de rétinol hydrogéné, les dits liposomes sont distribués de façon homogène dans une matrice de gel constituée d'eau et d'agent gélifiant, et le gel, ensemble avec des additifs, des substances auxiliaires, des principes actifs cosmétiques usuels et des mélanges d'entre eux, est inclus à une concentration de 0,1 - 11 % en poids dans la préparation cosmétique, la concentration se rapportant au poids total de la préparation.

2. Préparation cosmétique selon la revendication 1, dans laquelle l'extrait de graines de Plukenetia volubilis est dans une gamme de 1 à 3 % en poids, rapporté au poids total du complexe.

3. Préparation cosmétique selon la revendication 1, dans laquelle l'extrait de feuilles de Cynara scolymus est dans une gamme de 2 à 4 % en poids, rapporté au poids total du complexe.

4. Préparation cosmétique selon la revendication 1, dans laquelle la teneur en rétinol hydrogéné est dans une gamme de 0,1 à 1 % en poids, rapporté au poids total du complexe.

5. Préparation cosmétique selon la revendication 1, dans laquelle la teneur en complexe est dans une gamme de 1 à 6 % en poids, rapporté au poids total de la préparation.

6. Préparation cosmétique selon la revendication 1, **caractérisée en ce que** qu'elle contient des acides gras oméga-3/-6 à titres d'autres principes actifs.

7. Procédé de fabrication d'une préparation cosmétique comprenant un complexe de soins de la peau ayant un effet anti-vieillissement, **caractérisé en ce qu'**on
a) mélange une ou plusieurs huiles ainsi que des principes actifs extrait d'Inca inchi, extrait d'artichaut et le rétinol hydrogéné avec de l'alcool et de l'eau à 15 - 25 °C pour constituer un premier mélange, ensuite on
b) ajoute le premier mélange préparé dans a) à un second mélange préparé séparément, à une vitesse d'ajout non supérieure à 10 g/min à 1000 - 3000 t/min, moyennant quoi le second mélange est constitué d'un phospholipide et d'autres substances auxiliaires et principes actifs,
c) on agite le troisième mélange ainsi obtenu à 600 - 800 t/min jusqu'à formation des liposomes,
d) on réunit l'eau et l'agent gélifiant et on agite à une vitesse non supérieure à 1200 t/min jusqu'à formation d'une matrice de gel,
e) on introduit le troisième mélange à une vitesse inférieure à 700 t/min pendant une période de 15 - 40 min dans le gel selon l'étape d), et
f) on mélange le produit obtenu selon e) à 20-35 °C et inférieure à 700 t/min avec des substances auxiliaires et principes actifs cosmétiques usuels ou des mélanges d'entre eux.
